# EUROPEAN PATENT APPLICATION

(11) **EP 0 832 892 A1**
(43) Date of publication of application: **01.04.1998**
(21) Application number: 97202885.6
(22) Date of filing: 19.09.1997
(51) Int. Cl.: C07D 501/00, C07D 501/12, C12P 35/00

(54) **Process for purifying cephalexin**

(30) Priority: 27.09.1996 BE 9600813
(71) Applicant: CHEMFERM V.O.F., 4817 HX Breda (NL)
(72) Inventor: Van Dooren, Theodorus Johannes Godfried Maria, 6042 BZ Roermond (NL); Boesten, Wilhelmus Hubertus Joseph, 6132 BJ Sittard (NL)
(74) Representative: Jacobs, Monique S.N.

(57) **Abstract**

Process for removing impurities, for instance D-phenylglycine amide, 7-aminodesacetoxycephalosporanic acid and proteins from solid cephalexin containing such impurities , in which the solid cephalexin is dissolved and is subjected to a treatment with active carbon. Preferably the cephalexin solution that is subjected to the treatment with active carbon is a saturated cephalexin solution; and the treatment with active carbon takes place at a pH of between 6.5 and 9 and at a temperature of between 5 and 25°C.

## Description

The invention relates to a process for removing D-phenylglycine amide or a D-pheylglycine esters and/or 7-aminodesacetoxy-cephalosporanic acid and optionally proteins from solid cephalexin containing such impurities.

It is known to prepare cephalexin (CEX) via (enzymatic) acylation of 7-aminodesacetoxycephalosporanic acid (7-ADCA) with the aid of D-phenylglycine amide (PGA) or a D-phenylglycine ester, particularly D-phenylglycine methyl ester. Such a process is for example described in WO-A-93/12250. It has however been found that the cephalexin that is obtained after precipitation generally contains relatively large amounts of PGA and 7-ADCA and, optionally, proteins. In practice the cephalexin obtained often contains between 0.1 and 3.0 wt.% PGA and between 0.5 and 2.0 wt.% 7-ADCA, relative to the amount of cephalexin. The presence of such amounts of impurities in the cephalexin antibiotic is undesired.

The aim of the invention is to provide a process with which the impurities present in cephalexin can essentially be removed.

This is achieved according to the invention by dissolving the solid cephalexin and subjecting it to a treatment with active carbon.

Surprisingly, it has been found that PGA and/or 7-ADCA are removed from the solution during the treatment with carbon, whereas the addition of active carbon to the reaction mixture obtained after the acylation of 7-ADCA with PGA does not result in a significant reduction of the PGA and 7-ADCA contents of the solid cephalexin obtained after precipitation.

Surprisingly, the treatment with carbon also proves to be most useful as a method for removing residual protein from the cephalexin. Protein dyeing methods such as Bradford's determination, Anal. Biochem. 1976, 72, 248-254, show that a treatment with carbon results in a significant decrease of the protein content. The method is effective for the removal of protein already when the reaction mixture obtained after the acylation of 7-ADCA with PGA is treated as such with carbon.

The use of active carbon as a purifier is known, in particular in the removal of conjugated substances such as polyaromatic substances. Surprisingly, it has now been found that e.g. PGA is removed practically quantitatively and highly selectively, relative to the cephalexin, without significant cephalexin losses. A large portion of the 7-ADCA and the proteins can also be removed in this way. Preferably, in order to prevent cephalexin losses, the cephalexin is dissolved so that a saturated cephalexin solution is obtained.

In principle, any type of active carbon can be used in the process according to the invention. Suitable types of active carbon are for example commercially available as standard products. Examples of suitable active carbons are steam-activated carbon, for example Brilloniet, Norit®SX and Norit®SA, and chemically activated carbon, for example Norit®CA and Norit®CX.

The active carbon containing PGA and 7-ADCA and optionally proteins can be removed from the solution in a known manner, for example via filtration through a G4 filter (= approx. 10 µm pore size). The carbon can also be used as column material, with the solution to be treated being passed through the column.

The temperature at which the treatment with active carbon takes place is not particularly critical and lies for example between -5 and 35°C, preferably between -2 and 30°C, in particular between 0 and 25°C. The pH at which the treatment with active carbon is preferably carried out is essentially determined by the stability and solubility of the cephalexin. The treatment can take place in both an acid and a basic medium. In practice, the pH will usually lie either between 5.5 and 10, preferably between 6.0 and 9, or in the pH range between 0 and 3, preferably between 0.5 and 2.5.

After the treatment with active carbon, the cephalexin can be recovered in commonly known ways, for example through evaporation, reduction of the temperature or pH shifts.

The invention will now be further elucidated with reference to the examples without however being limited thereto.

### Example I

### Selective removal of PGA and 7-ADCA from cephalexin

In this experiment use was made of a composition that contained the following components (PG=D-phenylglycine):
- CEX: 95.7%
- 7-ADCA: 0.07%
- PG: 0.01%
- PGA: 0.16%

100 g of cephalexin of the above composition (i.e. containing 0.16% PGA) was suspended in 900 g of water. Drops of a 25% ammonia solution in water were added to this until the pH was 8.1; this led to the formation of a clear solution. 5 g of Brilloniet was added to this solution. This was followed by 1.5 hours' stirring at room temperature.

The mixture was filtered through a G4 glass filter. Next, the mixture was in 1 hour dosed to a solution of 2 g of pure cephalexin in 100 g of water, which had previously been brought to a pH of 7 with the aid of a 25% aqueous ammonia solution. During the dosage of the mixture the pH was kept at 7 with the aid of 6N aqueous sulphuric acid.

The slurry obtained was stirred for another 0.5 hour and then filtered through a G3 glass filter. The cake was washed (3 x 100 ml of acetone) and dried, which resulted in 76.7 g of solid substance. The composition of the solid substance was:
- CEX:: 95.2%
- ADCA:: 0.02%
- PG:: 0.01%
- PGA:: 0.006%

The PGA content relative to the cephalexin had consequently decreased by a factor of 27. No PGA was detected in the mother liquid (it had hence all been adsorbed onto the Brilloniet); the mother liquid did contain approx. 20 g of cephalexin.

The 7-ADCA content relative to the cephalexin had also decreased, by a factor of 3.5.

### Example II

### Removal of residual protein from a cephalexin-containing stream

A solution was prepared that had the following composition:
50 g of cephalexin
25 g of 7-ADCA
7.5 g of PGA
2 g of PG
440 g of demineralised water.

The pH was set to 8.5, which resulted in the formation of a clear solution. The A600 was determined with the aid of Bradford's determination; it amounted to 0.609 (= blank value). Next, 0.4 ml of acylase protein was added to 100 g of this solution. A600/Bradford: 0.905.

Then 1 g of Brilloniet was added and the mixture was stirred for 2 hours at room temperature, after which it was filtered through a G-4 glass filter. A600/Bradford: 0.762. This value indicates that approx. 50% of the added protein had been removed.

## Claims

1. Process for the removal of D-phenylglycine amide or D-phenylglycine ester and/or 7-aminodesacetoxycephalosporanic acid and optionally proteins from solid cephalexin containing such impurities, characterised in that the solid cephalexin is dissolved and the solution obtained is subjected to a treatment with active carbon.

2. Process according to Claim 1, in which the solid cephalexin contains D-phenylglycine amide.

3. Process according to Claim 1 or 2, in which the cephalexin solution that is subjected to the treatment with active carbon is a saturated cephalexin solution.

4. Process according to any one Claims 1-3, in which the cephalexin is recovered in a solid form after the treatment with active carbon.

5. Process according to any one of Claims 1-4, in which the treatment with active carbon takes place at a pH of between 6.0 and 9.

6. Process according to any one of Claims 1-4, in which the treatment with active carbon takes place at a pH of between 0.5 and 2.5.

7. Process according to any one of Claims 1-6, in which the treatment with active carbon is carried out at a temperature of between 0 and 25°C.
